Europäisches Patentamt

**European Patent Office**

**Office européen des brevets**

(19)

(11) Veröffentlichungsnummer: **0 137 548**

**B1**

(12) **EUROPÄISCHE PATENTSCHRIFT**

(45) Veröffentlichungstag der Patentschrift:
**20.07.88**

(51) Int. Cl.⁴: **B 01 J 10/00,** C 07 C 51/31,
C 07 C 51/265, C 07 C 63/16

(21) Anmeldenummer: **84201307.0**

(22) Anmeldetag: **11.09.84**

(54) **Verfahren zum Vermischen einer fein zerteilten Flüssigkeit mit einem Gas und Erzeugen einer explosiven Mischung.**

(30) Priorität: **04.10.83 DE 3336022**

(43) Veröffentlichungstag der Anmeldung:
**17.04.85 Patentblatt 85/16**

(45) Bekanntmachung des Hinweises auf die Patenterteilung:
**20.07.88 Patentblatt 88/29**

(84) Benannte Vertragsstaaten:
**DE FR GB IT**

(56) Entgegenhaltungen:
**EP-A-0 052 745
DE-B-1 149 346
GB-A-2 006 039
US-A-2 453 740**

**CHEMICAL ENGINEERING PROGRESS, Band 66,
Nr. 9, September 1970, Seiten 49-53, New York, US;
R.F. SCHWAB et al.: "Hazards in Phthalic
Anhydride Plants"**

(73) Patentinhaber: **METALLGESELLSCHAFT AG,
Reuterweg 14 Postfach 3724, D-6000 Frankfurt/M.1
(DE)**

(72) Erfinder: **Geissen, Rolf, Ginnheimer Strasse 25,
D-6000 Frankfurt am Main (DE)**
Erfinder: **Siebert, Wolfgang, Stettenstrasse 30,
D-6000 Frankfurt am Main (DE)**

(74) Vertreter: **Rieger, Harald, Dr., Reuterweg 14,
D-6000 Frankfurt am Main (DE)**

**Beschreibung**

Die Erfindung betrifft ein Verfahren zum Vermischen einer fein zerteilten Flüssigkeit mit einem Gas, wobei für die Mischung beim Überschreiten einer Konzentrationsgrenze die Flüssigkeit (explosive Konzentration) in der Mischung Explosionsgefahr besteht, und Umsetzen der Mischung in einer Reaktionszone. Das Zerteilen der Flüssigkeit erfolgt z. B. durch Verdüsen oder Verdampfen.

Für die Durchführung derartiger Reaktionen empfiehlt sich eine möglichst gleichmäßige Verteilung der Ausgangskomponenten beim Eintritt in die Reaktionszone insbesondere dann, wenn die Umsetzung in Gegenwart eines Katalysators erfolgt. Deshalb hat man bisher die Vermischung der Ausgangskomponenten in großem Abstand vor der Reaktionszone vorgenommen, so daß durch Mischeinrichtungen und genügende Verweilzeit die gewünschte Homogenität der Mischung gewährleistet ist.

Bei Umsetzungen wie z. B. der Oxidation von Orthoxylol oder Naphthalin mit Luft zu Phthalsäureanhydrid ist im allgemeinen ein hoher Energieaufwand nötig, um das Gemisch auf den erforderlichen Druck in der Reaktionszone zu bringen. Deshalb ist man bestrebt, die Beladung des Gases mit der fein zerteilten Flüssigkeit zu erhöhen, um die Gesamt-Energiebilanz des Verfahrens zu verbessern. Hierbei ist es aber nicht einfach, eine gleichmäßige Verteilung der Flüssigkeit im Gas zu erreichen.

Dem Verfahren der Erfindung liegt die Aufgabe zugrunde, für gleichmäßige Verteilung zu sorgen und Explosionssicherungen, soweit nötig, für ein möglichst niedriges Apparatevolumen auslegen zu können. Erfindungsgemäß geschieht dies dadurch, daß man in das Gas in einer ersten Mischzone einen Teil der Flüssigkeit in einer solchen Menge zumischt, daß die explosive Konzentration nicht erreicht wird und in einer zweiten Mischzone, die der Reaktionszone unmittelbar vorgeschaltet ist, in die Mischung aus der ersten Mischzone den Restteil der Flüssigkeit zumischt, wobei die explosive Konzentration in der Mischung überschritten wird. Bei diesem Verfahren liegt das mit Explosionssicherungen zu versehende Apparatevolumen nur noch in der kurzen Leitungsstrecke zwischen der zweiten Mischzone und der Reaktionszone.

Zweckmäßigerweise gibt man den größeren Teil der insgesamt mit dem Gas zu vermischenden Flüssigkeit in die erste Mischzone, und der kleinere Restteil der Flüssigkeit wird in die zweite Mischzone geleitet.

Umsetzungen der eingangs genannten Art mit Explosionsgefahr sind vor allem Oxidationen, wobei es sich bei dem Gas um sauerstoffhaltiges Gas handelt. Will man z. B. Orthoxylol mit Luft umsetzen, um in der Reaktionszone katalytisch Phthalsäureanhydrid zu erzeugen, so liegt die explosive Konzentration des Orthoxylols etwa bei 47 g Orthoxylol pro $Nm^3$ Luft. Aus Gründen der Wirtschaftlichkeit ist man aber bestrebt, der Reaktionszone eine Mischung zuzuführen, die pro $Nm^3$ Luft mindestens 50 g Orthoxylol oder mehr und nach Möglichkeit mindestens 60 g Orthoxylol enthält. Hierbei enthält die Mischung, die aus der ersten Mischzone kommt, höchstens etwa 45 g Orthoxylol pro $Nm^3$ Luft. Für ein Gemisch aus Naphthalin und Luft liegt die Explosionsgrenze bei 45 g Naphthalin pro $Nm^3$ Luft.

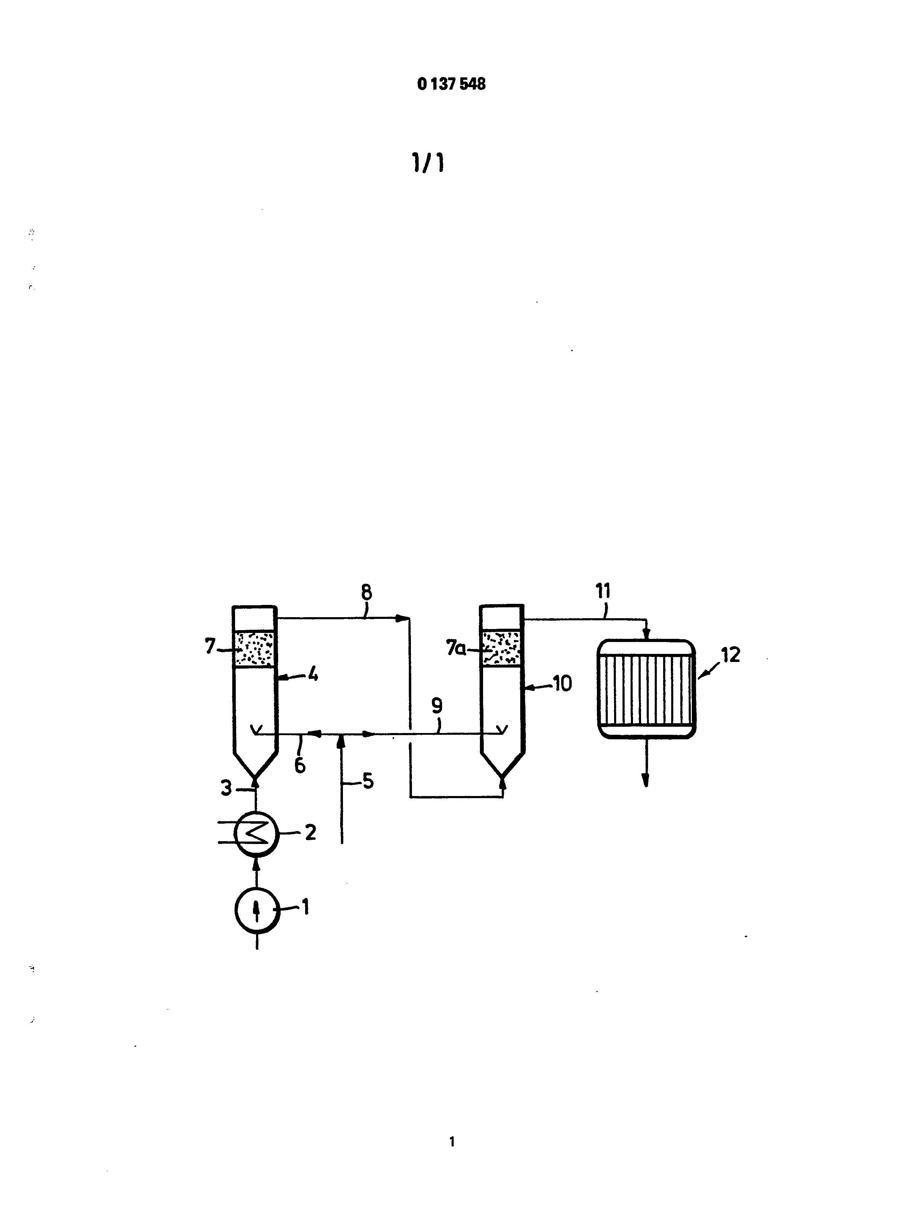

Ein Verfahrensbeispiel wird mit Hilfe der Zeichnung erläutert.

Durch das Gebläse 1 angesaugte Luft wird im wärmeaustauscher 2 auf eine Temperatur von etwa 180°C erwärmt und in der Leitung 3 einer ersten Mischzone 4 zugeführt. Gleichzeitig wird Orthoxylol aus der Leitung 5 zum größeren Teil durch die Zweigleitung 6 der Mischzone 4 zugeführt und dort fein verdüst. Die noch inhomogene Mischung aus Flüssigkeit und Gas strömt durch eine Verwirbelungsschicht 7, die aus Füllkörpern oder z. B. Streckmetall besteht. Durch die Leitung 8 verläßt eine Mischung mit 45 g Orthoxylol pro $Nm^3$ Luft die erste Mischzone 4 und wird der zweiten Mischzone 10 zugeführt.

Die Leitung 8 muß eine ausreichende Länge von mehreren Metern aufweisen, da die homogene Verteilung der Flüssigkeit im Gas erst beim Durchströmen durch diese Leitung erreicht wird. Durch die Zweigleitung 9 wird der zweiten Mischzone der kleinere Restteil des Orthoxylols aufgegeben und ebenfalls fein verdüst. Die zweite Mischzone weist ebenso wie die erste Mischzone eine Verwirbelungsschicht 7a auf. Die homogene Mischung, in welcher die explosive Konzentration überschritten ist, gelangt durch die Leitung 11 in den Reaktor 12, in welchem Phthalsäureanhydrid erzeugt wird.

Wenn man anstelle von Orthoxylol Naphthalin in der gleichen Verfahrensanordnung und zum Erzeugen des gleichen Endproduktes verwendet, erzeugt man in der Mischzone 4, der man verdampftes Naphthalin durch die Leitungen 5 und 6 und Luft durch die Leitung 3 zuführt, zunächst ein Gemisch mit 43 bis 44 g Naphthalin pro $Nm^3$. Weiteres verdampftes Naphthalin wird durch die Leitung 9 dem Gemisch zugeführt. Die Mischung in der Leitung 11 enthält üblicherweise pro $Nm^3$ Luft mindestens 50 g Naphthalin oder Orthoxylol.

Die Explosionssicherungen sind zweckmäßigerweise im oberen Bereich der Mischzone 10 und des Reaktors 12 angebracht. Diese Sicherungen können z. B. als Berstscheiben oder Knickstabsicherungen ausgebildet sein. Für die Umsetzung von Naphthalin oder Orthoxylol mit Luft enthält der Reaktor 12 einen in Röhren angeordneten bekannten Festbett-Katalysator mit $V_2O_5$ als Hauptbestandteil. Die Temperatur im Reaktor liegt bei etwa 360 bis 400°C, die Wärme der exothermen Reaktion wird indirekt durch ein Salzbad abgeführt.

## Patentansprüche

1. Verfahren zum Vermischen einer fein zerteilten Flüssigkeit mit einem Gas, wobei für die Mischung beim Überschreiten einer Konzentrationsgrenze für die Flüssigkeit (explosive Konzentration) in der Mischung Explosionsgefahr besteht, und Umsetzen der Mischung in einer Reaktionszone, dadurch gekennzeichnet, daß man in das Gas in einer ersten Mischzone einen Teil der Flüssigkeit in einer solchen Menge zugibt, daß die explosive Konzentration nicht erreicht wird, und in einer zweiten Mischzone, die der Reaktionszone unmittelbar vorgeschaltet ist, in die Mischung aus der ersten Mischzone den Restteil der Flüssigkeit zugibt, wobei die explosive Konzentration in der Mischung überschritten wird.

2. Verfahren nach Anspruch 1, dadurch gekennzeichnet, daß es sich bei dem Gas um sauerstoffhaltiges Gas handelt und in der Reaktionszone eine Oxidation erfolgt.

3. Verfahren nach Anspruch 2, dadurch gekennzeichnet, daß es sich bei der Flüssigkeit um Orthoxylol oder Naphthalin und beim Gas um Luft handelt, wobei die Mischung aus der zweiten Mischzone mindestens 50 g Orthoxylol oder Naphthalin pro Nm³ Luft enthält und in der Reaktionszone katalytisch zu Phthalsäureanhydrid umgesetzt wird.

4. Verfahren nach Anspruch 3, dadurch gekennzeichnet, daß die in der ersten Mischzone erzeugte Mischung pro Nm³ Luft höchstens etwa 45 g Orthoxylol oder höchstens 44 g Naphthalin enthält.

## Claims

1. Process for mixing a finely divided liquid with a gas, where there is a danger of explosion for the mixture if a concentration limit for the liquid in the mixture (explosive concentration) is exceeded and conversion of the mixture in a reaction zone, characterised by the fact that part of the liquid is added to the gas in a first mixing zone in such a quantity that the explosive concentration is not reached and that in a second mixing zone immediately preceding the reaction zone, the remainder of the liquid is added to the mixture from the first mixing zone.

2. Process according to Claim 1, characterised by the fact that the gas is an oxygen-containing gas and that oxidation occurs in the reaction zone.

3. Process according to Claim 2, characterised by the fact that the liquid is orthoxylene or naphthalene and the gas is air, where the mixture from the second mixing zone contains at least 50 g of orthoxylene or naphthalene per Nm³ of air and is converted catalytically in the reaction zone to phthalic anhydride.

4. Process according to Claim 3, characterised by the fact that the mixture produced in the first mixing zone contains a maximum of 45 g of orthoxylene or a maximum of 44 g of naphthalene per Nm³ of air.

## Revendications

1. Procédé de mélange d'un liquide à l'état finement divisé à un gaz, avec danger d'explosion pour le mélange si une limite de concentration du liquide (concentration explosive) dans le mélange est dépassée, et de réaction du mélange dans une zone de réaction, caractérisé en ce qu'il consiste à ajouter, dans une première zone de mélange, une partie du liquide en une quantité telle que la concentration explosive n'est pas atteinte, et à ajouter, dans une seconde zone de mélange qui est prévue immédiatemnt en amont de la zone de réaction, au mélange provenant de la première zone de mélange, la partie restante du liquide, la concentration explosive étant dépassée dans le mélange.

2. Procédé suivant la revendication 1, caractérisé en ce que le gaz est un gaz contenant de l'oxygène et il s'effectue une oxydation dans la zone de réaction.

3. Procédé suivant la revendication 2, caractérisé en ce que le liquide est l'orthoxylène ou le naphtalène, et le gaz est de l'air, le mélange provenant de la seconde zone de mélange contenant au moins 50 g d'orthoxylène ou de naphtalène par m³ normal d'air, et étant transforms catalytiquement en anhydride phtalique dans la zone de reaction.

4. Procédé suivant la revendication 3, caractérisé en ce que le mélange produit dans la première zone de réaction contient, par m³ normal d'air, au plus 45 g environ d'orthoxylène ou au plus 44 g de naphtalène.